(19)

Europäisches
Patentamt
European
Patent Office
Office européen
des brevets

(11) **EP 4 116 436 A1**

(12) **EUROPEAN PATENT APPLICATION**
published in accordance with Art. 153(4) EPC

(43) Date of publication:
**11.01.2023 Bulletin 2023/02**

(21) Application number: **21738505.3**

(22) Date of filing: **06.01.2021**

(51) International Patent Classification (IPC):
**C12Q 1/6886** $^{(2018.01)}$     **G16B 35/10** $^{(2019.01)}$

(52) Cooperative Patent Classification (CPC):
**C12Q 1/6886; G16B 35/10**

(86) International application number:
**PCT/KR2021/000116**

(87) International publication number:
**WO 2021/141374 (15.07.2021 Gazette 2021/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.01.2020   KR 20200002260**

(71) Applicants:
• **Korea Advanced Institute of Science and
Technology
Daejeon 34141 (KR)**
• **Pentamedix Co., Ltd.
Gyeonggi-do 13449 (KR)**

(72) Inventors:
• **CHOI, Jung Kyoon
Daejeon 34141 (KR)**

• **BANG, Hyo Eun
Daejeon 34141 (KR)**
• **PARK, Jae Soon
Daejeon 34141 (KR)**
• **CHO, Dae Yeon
Seongnam-si Gyeonggi-do 13540 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

Remarks:
The complete document including Reference
Table(s) and the Sequence Listing(s) can be
downloaded from the EPO website

(54) **METHOD AND SYSTEM FOR SCREENING FOR NEOANTIGENS, AND USES THEREOF**

(57)     Provided are a method and system for screening neoantigen and uses of neoantigens. Specifically, provided are a method and system for screening neoantigens derived from a gene of which expression is essential for survival of a cancer cell and/or a is homogeneously expressed in all cells in cancer tissue as a diagnostic and/or therapeutic target, and uses of neoantigens.

FIG. 1

EP 4 116 436 A1

**Description**

TECHNICAL FIELD

The present disclosure generally relates to method and system for screening neoantigens and uses thereof. In particular, the present disclosure relates to method and system for screening neoantigens derived from a gene of which expression is essential for survival of a cancer cell and/or is homogeneously expressed in all cells in cancer tissue as a diagnostic and/or therapeutic target, and uses of neoantigens.

BACKGROUND ART

Anticancer immunotherapy is currently drawing the most attention as an anticancer therapy and is deemed to present a new paradigm for anticancer therapy as high cure rates can be expected in a group of responsive patients. Studies including over 3,400 global immunotherapy clinical trials are actively being conducted worldwide.

An anti-cancer vaccine is a type of immunotherapy drug that activates the immune system by using a cancer-specific antigen, and is emerging as a core technology for anticancer immunotherapy based on its combination with an immune checkpoint blockade. However, the actual success rate of the checkpoint blockade, for example, anti-PD-1/anti-PD-L1, is only about 15 % to about 20 %, and the checkpoint blockade involves loss of general immunomodulatory functions of T cells, and thus there is a high risk of side effects such as autoimmune diseases. Accordingly, there are still needs for a therapeutic agent that can improve the response rate of cancer patients and minimize side effects thereof.

A patient-specific anti-cancer vaccine can be designed as an optimal vaccine targeting a patient-specific neoantigen, and thus is developed as a treatment method that can enhance therapeutic effects and minimize side effects for cancer patients. In this regard, the key is to identify optimal neoantigens so that immune action in a patient is induced to focus on a cancer cell-specific neoantigen to enhance anticancer effects of immunotherapy.

cancer cells evolve into a form that can evade an anticancer immune response through a process called immunoediting. A population of cancer cells evolveed in this way is considered to be the cause of resistance to anticancer immunotherapy and cancer recurrence. Accordingly, it is required to develop neoantigen targets that can overcome such an immune evasion attributable to the heterogeneity and plasticity of tumors. Korean Patent Application Laid-Open No. 2018-0107102 discloses methods of identifying and selecting neoantigens for a personalized cancer vaccine, but does not disclose or suggest the need for overcoming the immune evasion of cancer cells as well as the immune evasion of cancer cells.

The inventors of the present disclosure have tried to develop strategies to evade immunoediting mechanisms of cancer cells that make it difficult to discover an effective neoantigen as an anti-cancer vaccine, thereby completing the present disclosure related to a method of screening neoantigens as an effective diagnostic/therapeutic target.

DESCRIPTION OF EMBODIMENTS

TECHNICAL PROBLEM

The present disclosure aims to provide a method of screening neoantigens that make it possible to overcome immune evasion of cancer cells.

The present disclosure further aims to provide a system for screening neoantigens that make it possible toovercome immune evasion of cancer cells.

The present disclosure further aims to provide a anti-cancer vaccine including neoantigens that make it possible to overcome immune evasion of cancer cells.

The present disclosure further aims to provide a composition for predicting treatment prognosis of a cancer patient, comprising neoantigens that make it possible to overcome immune evasion of cancer cells.

SOLUTION TO PROBLEM

An aspect of the present disclosure provides a method of screening neoantigens, the method including:

> obtaining sequencing data of an exome, a transcriptome, a single cell transcriptome, a peptidome, an entire genome from a cancer patient;
> identifying genes essential for cancer cell survival; and
> obtaining neoantigens derived from the genes essential forcancer cell survival.

In an embodiment of the present disclosure, the method of screening neoantigens may further include determining binding affinity of the neoantigens to HLA of an antigen-presenting cell.

In an embodiment of the present disclosure, the gene essential for cancer cell survival may cause apoptosis various types of cancer cells or apoptosis of cancer cells derived from a cancer patient, when an expression level of the gene essential for cancer cell survival is reduced or is removed.

In an embodiment of the present disclosure, the gene essential for cancer cell survival may be a universal dependency gene, which is essential for survival of various types of cancers or cancer cells.

In an embodiment of the present disclosure, the gene essential for cancer cell survival may be a cancer patient-specific dependency gene, which is essential for survival of cancer cells derived from the cancer patient.

In an embodiment of the present disclosure, the gene essential for cancer cell survival may include a universal dependency gene or a cancer patient-specific dependency gene, or both a universal dependency gene and a cancer patient-specific dependency gene.

In an embodiment of the present disclosure, the identifying of the gene essential for cancer cell survival may include identifying the gene essential for cancer cell survival by using a model for predicting cell survival dependency, wherein the model for predicting cell survival dependence may be generated by learning a relationship between gene expression pattern in a cell and cell apoptosis, and identify as the gene essential for cancer cell survival a gene of which expression reduction or removal can cause cancer cell.

In an embodiment of the present disclosure, the model for predicting cell survival dependence may be based on deep learning of experimental data on the relationship between gene expression in a cell and cell apoptosis.

In an embodiment of the present disclosure, the relationship between gene expression in a cell and cell apoptosis may be based on *in vitro* screening experimental data or *in silico* data on whether a cancer cell undergoes apoptosis according to a reduction in expression or removal of a targeted gene.

In an embodiment of the present disclosure, the identifying of the gene essential for cancer cell survival may further include determining whether the gene essential for cancer cell survival identified by using the model for predicting cell survival dependence is homogeneously expressed in all cancer cells obtained from a cancer patient.

In an embodiment of the present disclosure, the obtaining of the neoantigens derived from the gene essential for cancer cell survival may include obtaining neoantigens of a cancer patient by comparing a cancer cell and a normal cell based on the sequencing data obtained from the cancer patient and collecting neoantigens derived from the obtained gene essential for cancer cell survival.

In an embodiment of the present disclosure, the obtaining of the neoantigens derived from the gene essential for cancer cell survival may include obtaining neoantigens of a cancer patient by comparing a sequence from the sequencing data obtained from a cancer patient and a sequence from the sequencing data obtained from a normal control group and collecting neoantigens derived from the obtained gene essential for cancer cell survival is dependent.

In an embodiment of the present disclosure, the collecting of the neoantigens may further include selecting nonsynonymous mutations in the gene essential for cancer cell survival.

In an embodiment of the present disclosure, the determining of the binding affinity of the neoantigens to the HLA of the antigen-presenting cell may include obtaining prediction of the binding affinity by inputting a sequence of the neoantigen to a model for predicting binding affinity of a peptide to HAL of an antigen-presenting cell, wherein the model for predicting binding affinity of a peptide may be generated by learning data regarding interaction between amino acids of peptides and amino acids of the HAL.

In an embodiment of the present disclosure, the antigen-presenting cell may include a dendritic cell, a macrophage, a B cell, or a combination thereof.

In an embodiment of the present disclosure, the HLA may be major histocompatibility class (MHC) I or MCH II.

In an embodiment of the present disclosure, when a CNN-MHC value between the neoantigen and the HAL of the antigen-presenting cell is > 0.5, the neoantigen is determined as having binding affinity.

In an embodiment of the present disclosure, more than one genes may be identified as the gene essential for cancer cell survival according to the essentiality for the survival of a cancer cell.

In an embodiment of the present disclosure, the top and bottom 5, 10, or more of the genes essential for cancer cell survival may be selected in terms of the essentiality for the cancer cell survival, respectively.

In an embodiment of the present disclosure, the neoantigen may be specific to a cancer patient.

Another aspect of the present disclosure provides a system for screening neoantigens, the system including:

a memory for storing at least one instruction; and
at least one processor executing the at least one instruction stored in the memory,
wherein the processor executes the at least one instruction
to generate a model for predicting cell survival dependency that predicts dependence of cell survival on gene expression by learning a relationship between a gene expression level in a cell and cell apoptosis,
to identify a gene essential for cancer cell survival by inputting a gene expression profile of a cancer patient to the model for predicting cell survival dependency,

to obtain the gene expression profile of the cancer patient a neoantigen derived from the gene essential for cancer cell survival,

to generate a model for predicting binding affinity of a neoantigen which predicts binding affinity based on amino acid interactions between a peptide and an antigen-presenting cell, and

to select a neoantigen having binding affinity to HLA of the antigen-presenting cell by using the model for predicting binding affinity of a neoantigen.

In an embodiment of the present disclosure, the system may be used to perform the method of screening the neoantigen according to an embodiment of the present disclosure.

In an embodiment of the present disclosure, the model for predicting cell survival dependency may identify a gene essential for cell survival or predict cell survival dependency of a gene from which a neoantigen is derived.

In an embodiment of the present disclosure, the model for predicting cell survival dependency may be generated by learning a relationship between a gene expression level in a cell and cell apoptosis, and may identify as a gene essential for cancer cell survival a gene of which expression reduction or removal causes cancer cell apoptosis.

In an embodiment of the present disclosure, the gene essential for cancer cell survival may be a universal dependency gene that is universally essential for survival of various types of cancers or cancer cells.

In an embodiment of the present disclosure, the gene essential for cancer cell survival may be a cancer patient-specific dependency gene essential for survival of a cancer cell derived from a specific cancer patient.

In an embodiment of the present disclosure, the gene essential for cancer cell survival may include a universal dependency gene or a cancer patient-specific dependency gene, or both a universal dependency gene and a cancer patient-specific dependency gene.

In an embodiment of the present disclosure, the processor may execute the at least one instruction to select a neoantigen having binding affinity, when a CNN-MHC value between the neoantigen and the HLA of the antigen-presenting cell is > 0.5.

In an embodiment of the present disclosure, the processor may execute the at least one instruction to learn the relationship between gene expression and cell apoptosis and the relationship of binding affinity between the neoantigen and the HLA of the antigen-presenting cell, respectively.

In an embodiment of the present disclosure, the learning may be performed based on deep learning.

In an embodiment of the present disclosure, the relationship between gene expression in a cell and cell apoptosis may be based on *in vitro* data or *in silico* data on whether a cancer cell line undergoes apoptosis according to a reduction in expression or removal of a targeted gene.

In an embodiment of the present disclosure, the model for predicting binding affinity of the neoantigen may be generated by learning data regarding interaction between amino acids of the peptide and amino acids of the HLA.

In an embodiment of the present disclosure, the gene expression profile of the cancer patient may be the sequencing data of an exome, a transcriptome, a single cell transcriptome, a peptidome, or an entire genome.

In an embodiment of the present disclosure, the HLA may be MHC class I or MHC class II.

In an embodiment of the present disclosure, when a CNN-MHC value between the neoantigen and the HAL of the antigen-presenting cell is > 0.5, the neoantigen may be determined as having binding affinity.

In an embodiment of the present disclosure, more than one genes may be identified as the gene essential for cancer cell survival according to the essentiality for the survival of a cancer cell.

Another aspect of the present disclosure provides a method of preparing an anti-cancer vaccine, the method including:

obtaining a neoantigen screened by the aforementioned screening method according to an aspect of the present disclosure; and

preparing an anti-cancer vaccine comprising the neoantigen.

In an embodiment of the present disclosure, the method of preparing an anti-cancer vaccine may further include: obtaining peptide sequences including the neoantigen, the peptide sequences consisting of 9 to 30 amino acids; and selecting a peptide sequence having hydrophilicity and stability from among the obtained peptide sequences.

In an embodiment of the present disclosure, the selected peptide sequence may have Kyte-Doolittle GRAVY < 0 and InstaIndex < 40.

In an embodiment of the present disclosure, the selected peptide sequence may consist of 12 to 30 amino acids, 15 to 30 amino acids, or 15 to 25 amino acids.

Another aspect of the present disclosure provides an anti-cancer vaccine including a neoantigen screened by the aforementioned screening method according to an aspect of the present disclosure.

The anti-cancer vaccine may induce a specific cytotoxic T cell response and/or a specific helper T cell response.

In an embodiment of the present disclosure, the anti-cancer vaccine may include a peptide including the neoantigen.

In an embodiment of the present disclosure, the peptide may have a length consisting of 15 to 30 amino acids, and may bind to the HLA of the antigen-presenting cell and activate a T cell specific to the neoantigen.

In an embodiment of the present disclosure, the anti-cancer vaccine may include peptides including at least two neoantigens.

In an embodiment of the present disclosure, the anti-cancer vaccine may further include an additional active ingredient, such as an anti-cancer drug, an additive, an excipient, and the like.

The anti-cancer vaccine may be administered in an amount sufficient to induce an antigen-specific immune response. The amount of the peptide to be included in the anti-cancer vaccine or a dosage of the anti-cancer vaccine may be determined by those skilled in the art without undue experimentation. The anti-cancer vaccine may be administered by intravenous injection, subcutaneous injection, intradermal injection, intraperitoneal injection, or intramuscular injection. A concentration of the peptide in the anti-cancer vaccine may vary such as less than about 0.1 wt%, about 2 wt% to about 20 wt%, and about 50 wt% or more, and may be determined in consideration of a treatment regime and the like. The dosage of the anti-cancer vaccine may be determined by a clinician in consideration of a composition of the peptide including the neoantigen, treatment regime, a stage and severity of a target disease, and a weight, health condition of a patient, and the like. The anti-cancer vaccine may be generally administered, in the case of a patient weighing 70 kg, in an amount of the peptide ranging about 1.0 μg to about 50,000 μg peptide.

Another aspect of the present disclosure provides a method of providing information to predict treatment prognosis of a cancer patient, the method including: obtaining a neoantigen obtained by the aforementioned screening method according to an aspect of the present disclosure; and
measuring the neoantigen load in a sample from a cancer patient.

In an embodiment of the present disclosure, the method of providing the information to predict treatment prognosis of the cancer patient may further include comparing the load of the obtained neoantigen and a load of a neoantigen obtained from a control group consisting of cancer patients for whom treatment prognosis have been confirmed.

In an embodiment of the present disclosure, the control group may consist of cancer patients confirmed to have good treatment prognosis or cancer patients confirmed to have poor treatment prognosis.

In an embodiment of the present disclosure, the neoantigen load may refer to the number of neoantigens.

In an embodiment of the present disclosure, when the load or number of the neoantigen in the sample from the cancer patient is greater than that of the neoantigen in a control group consisting of cancer patients with poor treatment prognosis, the treatment prognosis of the cancer patient may be predicted to be good.

In an embodiment of the present disclosure, when the load or number of the neoantigen in the sample from the cancer patient is smaller than that of the neoantigen in a control group consisting of cancer patients with good treatment prognosis, the treatment prognosis of the cancer patient may be predicted to be poor.

Another aspect of the present disclosure provides a composition for predicting treatment prognosis of a cancer patient, the composition including a neoantigen obtained by the aforementioned screening method according to an aspect of the present disclosure.

The composition for predicting treatment prognosis of a cancer according to an aspect of the present disclosure may further include an additional ingredient necessary for predicting the treatment prognosis of a cancer patient.

ADVANTAGEOUS EFFECTS OF DISCLOSURE

A method according to an embodiment of the present disclosure may provide screening of neoantigens derived from a universal dependency gene essential for cancer cell survival or a cancer patient-specific dependency gene essential for cancer cell survival as a target for diagnosis and/or treatment, thereby making it possible to develop a neoantigen-based anti-cancer vaccine having an enhanced immunotherapeutic effects on cancer cells without concern about immune evasion of cancer cells, and to predict treatment prognosis of a cancer patient.

BRIEF DESCRIPTION OF DRAWINGS

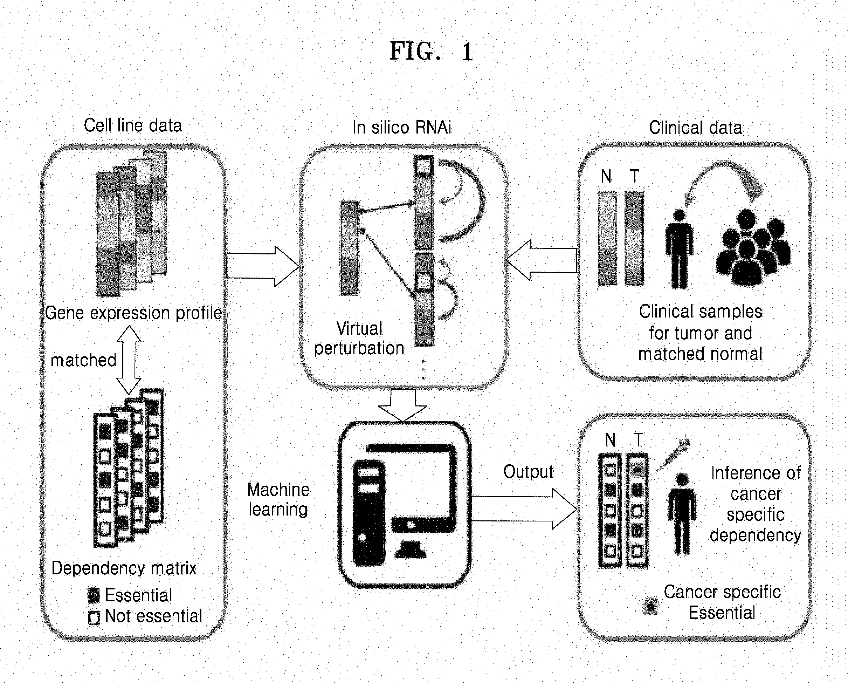

FIG. 1 is a schematic diagram illustrating an *in silico* method of predicting essentiality of a gene for cancer cell survival according to an embodiment of the present disclosure.

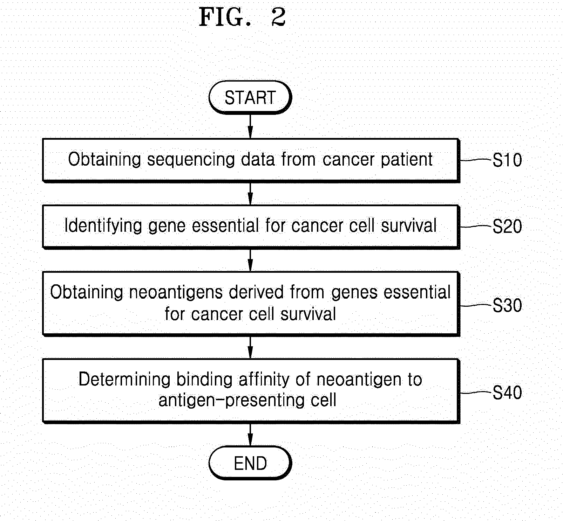

FIG. 2 is a flowchart illustrating a method of screening neoantigens according to an embodiment of the present disclosure. For the determination of the cancer cell survival dependency on the gene from which the neoantigen is derived and the determination of binding ability of the neoantigen to an antigen-presenting cell, the order and number thereof may be adjusted as necessary.

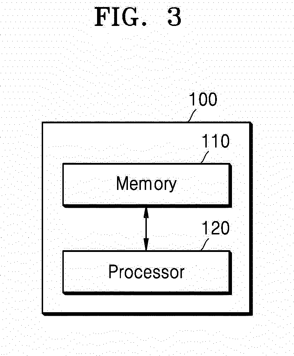

FIG. 3 is a block diagram illustrating a system for screening neoantigens according to an embodiment of the present disclosure.

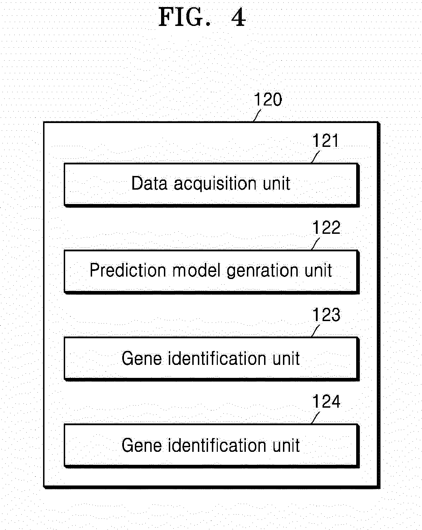

FIG. 4 is a block diagram of a processor according to an embodiment of the present invention.

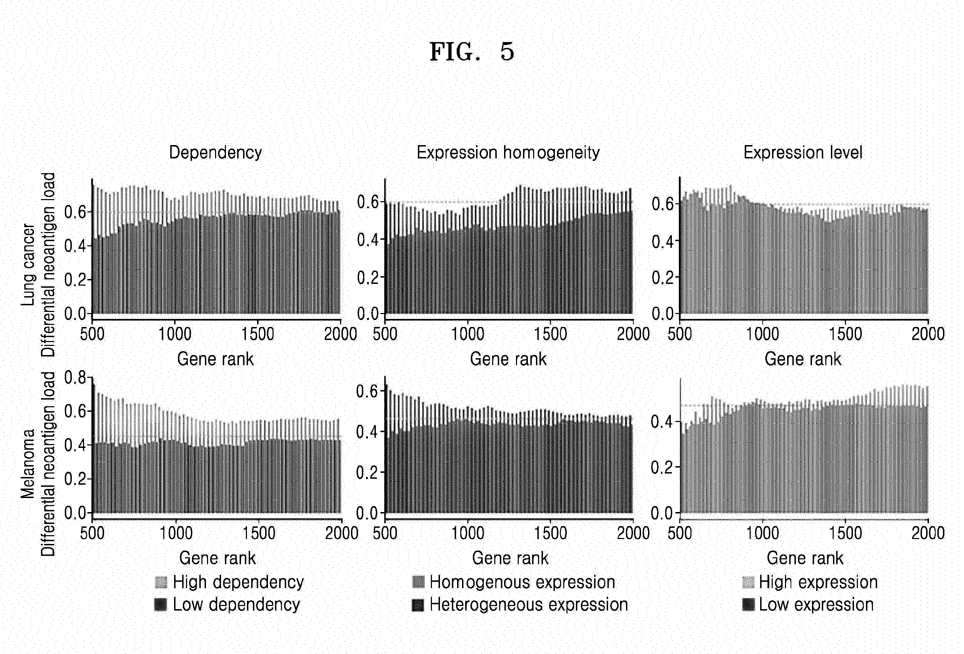

FIG. 5 shows significance of a neoantigen as a diagnostic and/or therapeutic target, the neoantigen obtained by the method of screening neoantigens using *in vitro* dependence data according to an embodiment of the present disclosure. By using the published *in vitro* dependency data and expression homogeneity data for lung cancer and melanoma cohorts, the usefulness of the method of screening neoantigens based on dependency of cancer call survival according to an embodiment, is verified. The gray dotted line indicates a difference in the number of neoantigens calculated for all genes according to an existing standard method.

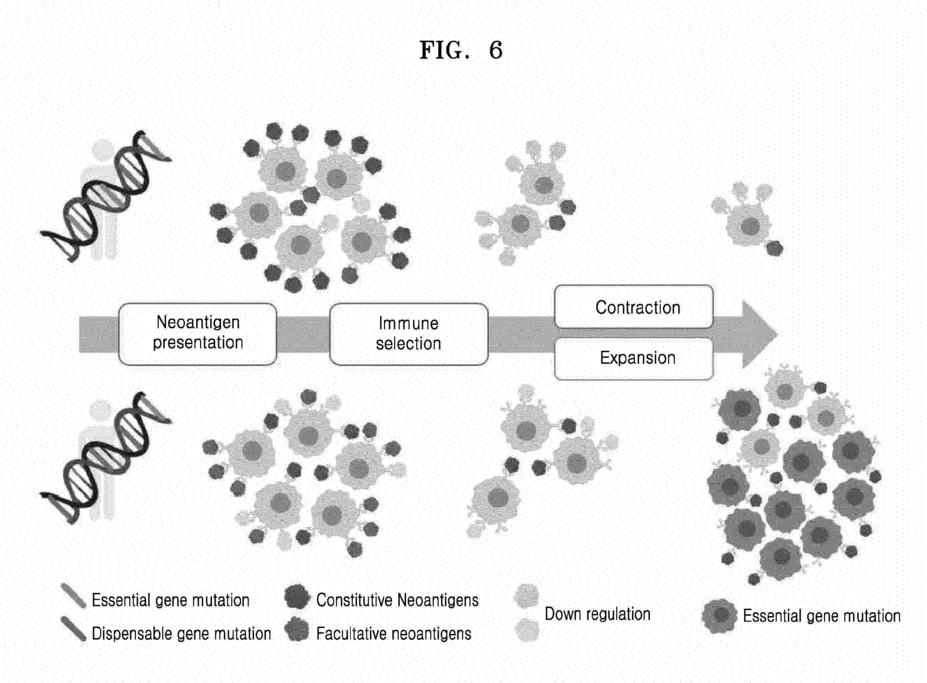

FIG. 6 is a schematic diagram of an immune evasion mechanism of cancer cells. It shows that when the subject neoantigen is a constitutive neoantigen derived from a protein which is essential for survival of cancer cells and is homogeneously expressed in all types of cells, the cancer cells effectively respond to immunotherapy (the upper panel), and that, when the subject neoantigen is a facultative neoantigen derived from a protein which is not essential to survival of cancer cells or is not homogeneously expressed in all types of cells, an immune evasion mechanism may occur against immunotherapy(the bottom panel).

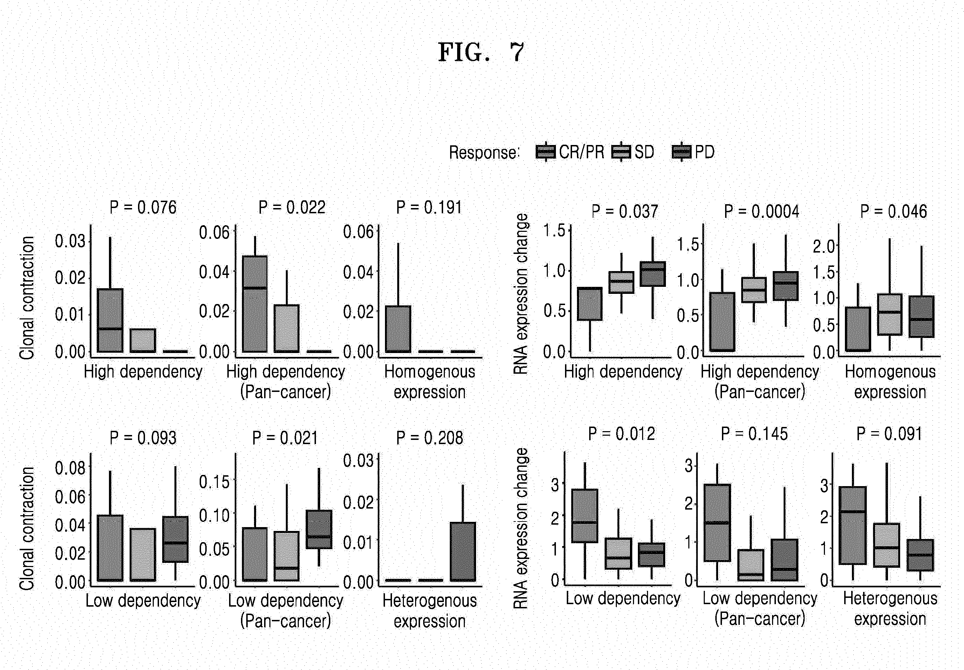

FIG. 7 shows patterns of response of neoantigens to immunotherapy, the neoantigens obtained by a method of screening neoantigens based on *in vitro* dependency data and single-cell gene expression data according to an embodiment of the present disclosure. Shown are results of comparing clonal changes and gene expression changes before and after treatment in a melanoma cohort prescribed with a checkpoint blockade (Riaz) depending on the essentiality of function of a gene from which the neoantigen is derived for cancer survival (high dependency vs. low dependency) and the homogeneity of gene expression (homogenous vs. heterogeneous expression). Here, CR/PR and SD/PD refer to positive prognosis and negative prognosis, respectively. In the case of patients with a positive prognosis, it is shown that neoantigens derived from high essentiality genes were mainly subjected to immune attack, leading to clonal contraction, and gene expression (RNA expression) reduction as a result of immunoediting. On the other hand, in the case of patients with a negative prognosis, it is shown that neoantigens derived from low essentiality genes are mainly subjected to immune attack, leading to clonal expansion, and gene expression reduction.

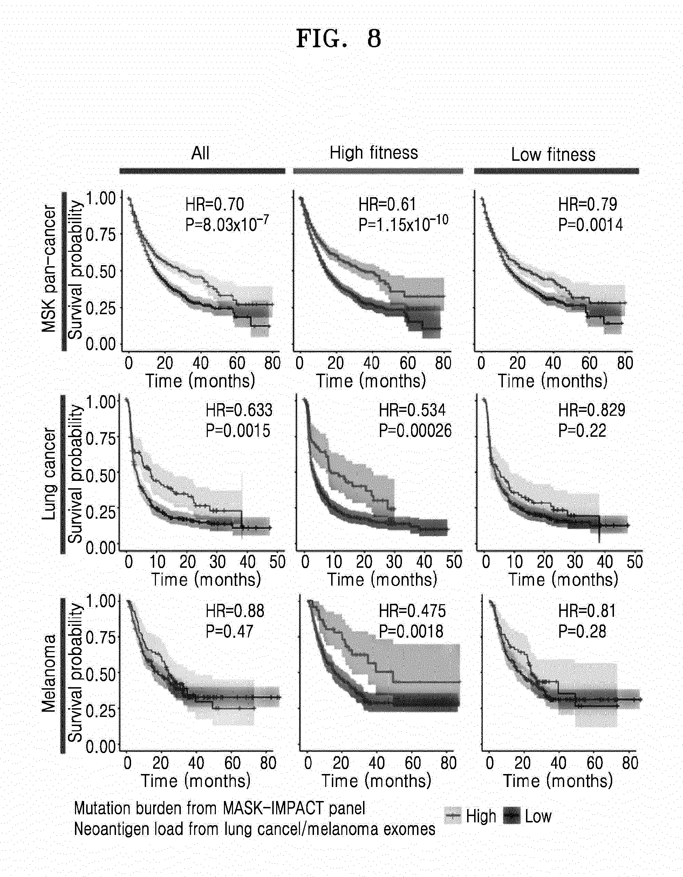

FIG. 8 shows significance of a neoantigen as a target for diagnosis and/or treatment, the neoantigen obtained by the method of screening neoantigens based on *in vitro* dependency data according to an embodiment of the present disclosure. Analysis similar to that shown in FIG. 5 was performed as survival analysis of MSKCC panel (468 genes) results for all types of cancer patient samples and lung cancer and melanoma immunotherapy cohorts. In FIG. 8, the upper panel (MSK pan-cancer) shows a result of analyzing mutant-derived genes for all of an MSKCC panel or the top 50 % essential genes (high fitness) or the bottom 50 % essential genes (low fitness). In FIG. 8, the middle panel (lung cancer) and the bottom panel (melanoma) show results of analyzing the published immunotherapy cohorts of lung cancer and melanoma for all genes or the top 500 genes (high fitness) and bottom 500 genes (low fitness). For each group of genes, comparisions of the post-treatment survival probability were shown for patients having a high mutation burden or neoantigen load (High) and those having a low mutation burden or neoantigen load (Low), along with hazard ratio (HR) values and p-values. The lower HR values and p-values indicate that the corresponding group of genes has high explanatory power with respect to the treatment prognosis. As a result, it was confirmed that the treatment prognosis was better explained by a small number of geness having high cancer cell survival dependency than all genes according to the standard method.

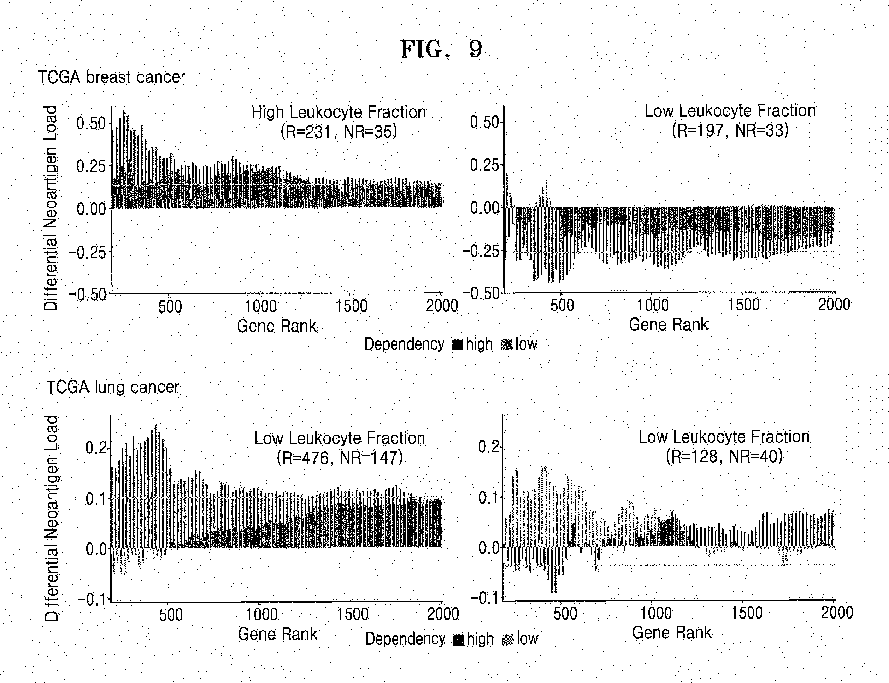

FIG. 9 shows significance of neoantigens as a diagnostic/therapeutic target, the neoantigens obtained based on cancer patient-specific *in silico* dependency data according to an embodiment of the present disclosure. Based on the transcriptome data for patients with lung cancer and breast cancer published by The Cancer Genome Atlas (TCGA) (https://portal.gdc.cancer.gov/), the *in silico* dependency prediction was performed as shown in FIG. 1. Since these patients did not actually receive the immunotherapy, the patients were divided into a sample having high penetration of immune cells (referred to as high leukocyte fraction) and a sample having low penetration of immune cells (referred to as low leukocyte fraction), assuming that an effect similar to immunotherapy would be found in the sample having high penetration of immune cells. As shown in FIG. 5, similar results are obtained only in samples having high penetration of immune cells by measuring the explanatory power of a gene from which a neoantigen is derived with respect to the survival a patient (where R indicates Responder; and NR indicates Nonresponder). The gray dotted line indicates a difference in the number of neoantigens calculated for all genes according to an existing standard method. Overall, the cancer patient-specific dependency data was found to a higher difference in terms of the explanatory power than the universal dependency data.

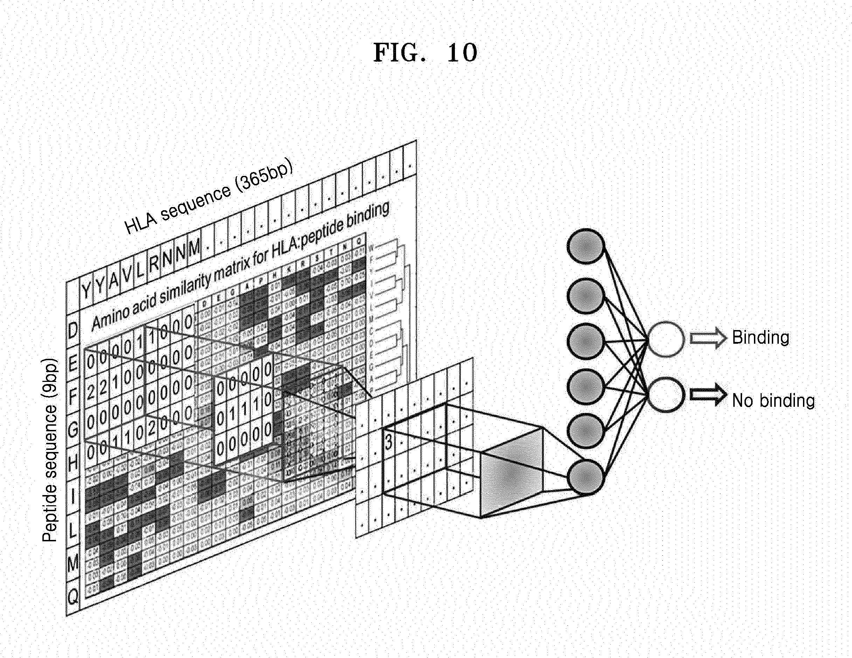

FIG. 10 is a schematic diagram of a model for predicting binding affinity of a neoantigen to MHC used in the neoantigen screening method according to an embodiment of the present disclosure. A model for predicting binding affinity between HLA and an antigenic peptide is shown in which a two-dimensional matrix between HLA and a neoantigenic peptide is established based on amino acid similarity matrix information, and a convolutional neural network (CNN) is applied thereto.

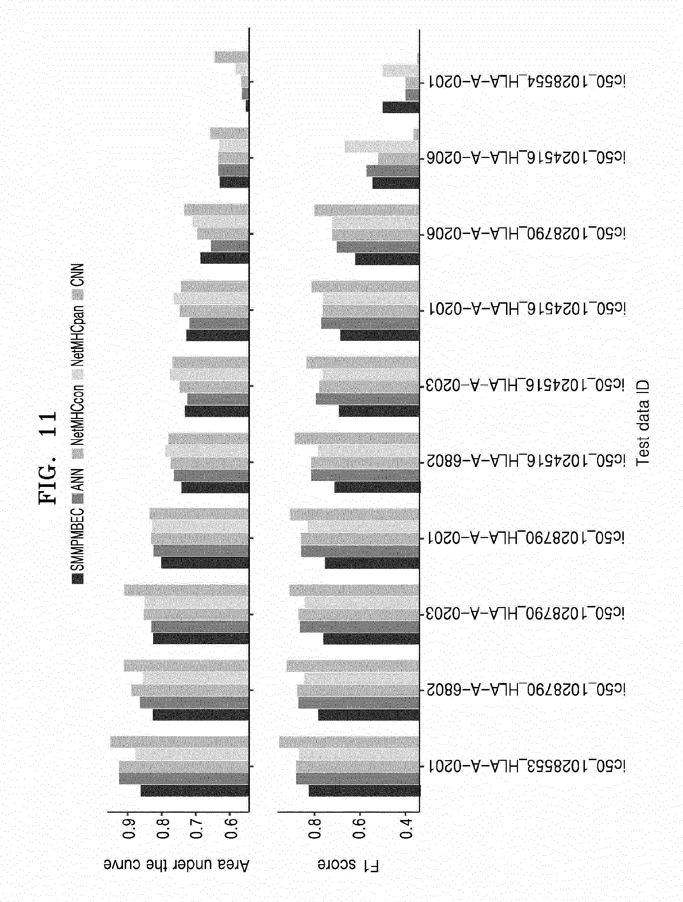

FIG. 11 shows comparison of a CNN according to an embodiment of the present disclosure and other methods (i.e. NetMHCpan, NetMHCcon, ANN, and SMMPMBEC) in performance (AUC and F1 score) by using a test data set officially provided by the Immune Epitope Database (IEDB).

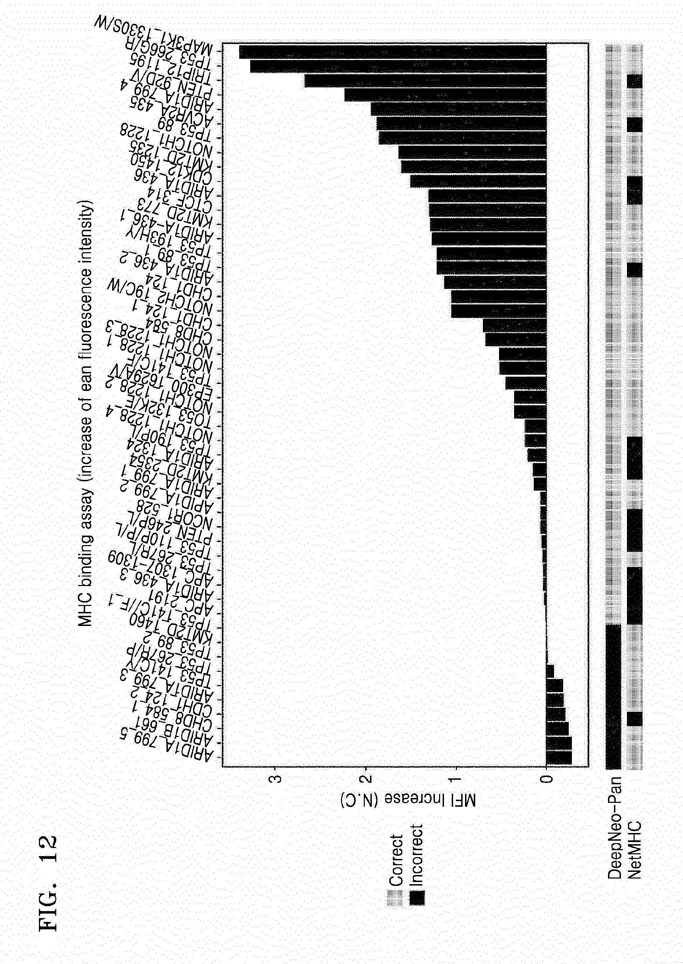

FIG. 12 shows the binding of neoantigens to HLA, as predicted according to an embodiment of the present disclosure. 80 % of peptides including neoantigens predicted to bind to HLA-A02 were found actually binding to HLA-A02.

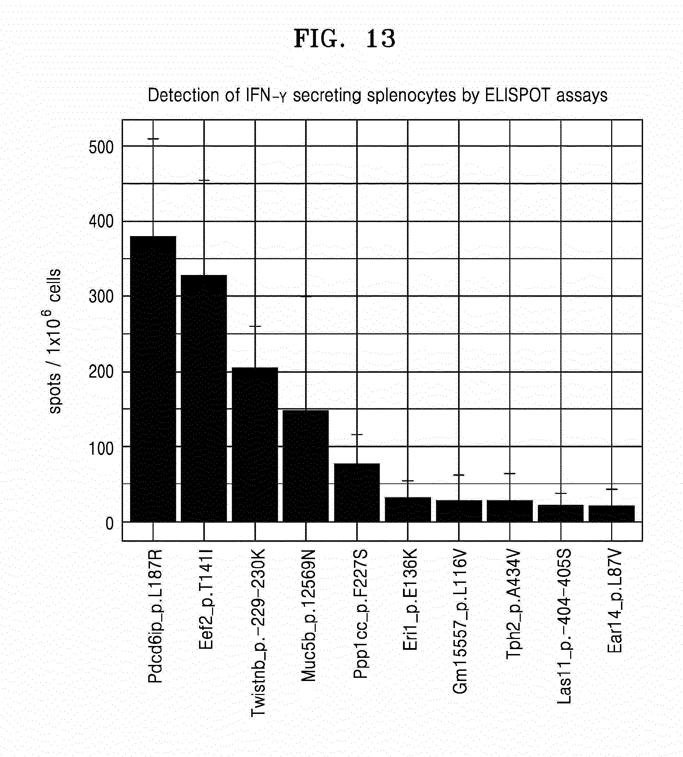

FIG. 13 shows immune responses induced by selected neoantigens according to an embodiment of the present disclosure. Regarding CD8+ T cell responses to neoantigens, that ELISpot analysis based on INFγ secretion showed that 10 (66.7 %) out of 15 candidate neoantigen peptides predicted to be reactive induced INFγ secretion by T cells.

MODE OF DISCLOSURE

The present disclosure will become apparent with reference to embodiments described in detail below in conjunction with the accompanying drawings. However, the present disclosure is not limited to embodiments below, but will be implemented in various forms. The present embodiments only serve to complete the disclosure of the present disclosure, and are provided to completely inform the scope of the disclosure to ordinary skill in the art to which the present disclosure pertains, and the present disclosure is defined by the claims.

The term "exome" as used herein refers to the collection of all of the exons present in a cell, a group of cells, or an organism.

The term "transcriptome" as used herein refers to the collection of all RNA transcripts present in a cell, a group of cells, or an organism.

The term "gene expression profile" as used herein refers to an analysis of genes expressed or transcribed from the genome of a cell, and also refers to a set of values indicating gene expression levels including the mRNA levels of one or more genes.

The term "dependency" as used herein refers to essentiality for proliferation or survival of a cell, and is used interchangeably with "essentiality".

The term "dependency gene" as used herein refers to a gene essential for proliferation or survival of a cell. In particular, the dependency gene is a gene that causes reduced cell proliferation and/or cell apoptosis when expression of the gene is reduced or the gene is removed, and that is, refers to a gene on which a cell depends for survival thereof. The dependency gene may include a universal dependency gene identified as universally essential for survival of cancers or cancer cells of various types and/or sources, and/or a cancer patient-specific dependency gene identified as specifically essential for survival of cancer cells derived from an individual cancer patient. The dependency gene may refer to a gene that is constitutively expressed in a cell and that is homogeneously expressed in all individual cells.

The term "universal dependency gene" as used herein refers to a gene identified as essential for survival of a range of various cancer cells through *in vitro* data of a known cancer cell line.

The term "cancer patient-specific dependency gene" as used herein refers to a gene identified as essential for survival of a cancer cell derived an individual cancer patient.

The term "neoantigen" as used herein refers to a peptide that causes an immune response. That is, the neoantigen may be an immunogenic peptide. The neoantigen may be generated by a cancer cell-specific mutation, and may appear as an epitope of a cancer cell. The neoantigen may be an antigen having at least one alteration that distinguishes it from a corresponding wild-type, parental antigen, either through mutation in a cancer cell or through post-translational modification specific to a cancer cell. The neoantigen may include an amino acid sequence or a nucleotide sequence. A mutation may include a frameshift or non-frameshift mutation, an indel, a missense or nonsense, a splice site mutation, a genomic rearrangement or gene fusion, or any genomic or expressional alteration resulting in a new ORF.

Since a neoantigen derived from a universal dependency gene or a cancer patient-specific dependency gene is not affected by immune evasion of cancer cells via immunoediting, such a neoantigen may be an effective therapeutic target for a cancer patient-personalized cancer vaccine that can have high immunotherapeutic effects in the cancer patient, and may be an effective diagnostic target as a marker for prognosis of immunotherapy.

The term "immunotherapy" as used herein refers to therapy using an immune response. Immunotherapy may be used to treat cancer. For example, the immunotherapy may be a treatment using an immune checkpoint blockade such as an anti-CTLA4 blocker or an anti-PD-1/PD-L1 blocker, but is not limited thereto, and may include various types of immunotherapy.

The term "binding affinity" as used herein refers to a binding force between a neoantigen peptide and MHC of an antigen-presenting cell, and may be expressed as a CNN-MHC value. The "CNN-MHC value" is a value obtained by establishing a deep learning model based on experimental values, the binding strength between respective amino acids of the neoantigen and the MHC converted into a matrix form, and means a probability value between 0 and 1 obtained by use of sigmoid activation function. Specifically, an immunogenic peptide capable of binding to an MHC Class I or II protein may have an MHC CNN-MHC level of greater than or equal to 0.5. In addition, as the CNN-MHC value gets close to 1, the binding between the immunogenic peptide and the MHC class I or II protein gets strong.

The term "an antigen-presenting cell" as used herein refers to a cell that internalizes and processes a protein antigen and presents on its surface an antigen-derived peptide fragment along with MHC class II to a T cell for activation, and examples thereof include a macrophage, a B cells, a dendritic cell, and the like.

The term "model for predicting cell survival dependency" as used herein refers to a model predicting the probability of cell survival or cell apoptosis in case of a reduction in expression or removal of an individual gene. Specifically, it is a model that predicts an effect of a specific gene on cell survival by learning the effect of knockout/knockdown of a gene on cell survival based on machine learning, and the machine learning may be performed based on *in vitro* data of the knockout/knockdown of a gene by RNAi or CRISPR/Cas9. When a gene expression profile or a sequence is input into the model for predicting cell survival dependency, cell survival dependency of a gene derived from the corresponding sequence may be predicted.

The term "model for predicting binding affinity of neoantigen" as used herein refers to a model predicting binding affinity of a neoantigen to an antigen-presenting cell, particularly, to MHC of an antigen-presenting cell. By learning the binding affinity resulting from the amino acid interactions between a peptide sequence and an HLA sequence based on the machine learning and inputting a peptide sequence of a neoantigen, the binding affinity of the neoantigen to the HLA of the antigen-presenting cell may be predicted, and the neoantigen may be classified according to the established scale of the binding affinity.

## Example 1. Selection of gene essential for cancer cell

To determine whether functions of a gene are essential for survival of cancer or cancer cells, high-throughput screening (HTS) can be performed by using an RNAi or CRISPR library capable of performing knockout/knockdown of all genes. Specifically, genes essential for survival of cancer cells can be identified *in vitro* by transfecting cancer cells with an shRNA library or CRISPR sgRNA, conducting deep sequencing after a certain period of time, and comparing the result with the sequencing result from the cells in the initial state to obtain quantitative profiling showing which genes, when inactivated, led to cell apoptosis. FIG. 1 schematically illustrates a method of predicting genes essential for survival of cancer cells.

In this way, *in vitro* data on genes essential for survival of cancer cells are continually being produced with an increasing number of cell lines. Accordingly, for major solid cancers, such as lung cancer, ovarian cancer, colorectal cancer, stomach cancer, breast cancer, and the like, dependency data on a significant number of cancer cell lines have been established (https://depmap.org/portal/ and https://depmap.sanger.ac.uk/). Then, according to an embodiment of the present disclosure, these data were used as data on universal dependency genes, or used for the purpose of predicting cancer patient-specific dependency genes *in silico* based on deep learning.

The *in vitro* data on the cancer cell lines can be used to obtain universal dependency genes, whereas the data derived from cancer patients, such as transcriptome data of cancer patients, can be used to identify a cancer patient-specific dependency gene by applying the data to a model for predicting cell survival dependency, which was trained based on the *in vitro* data on the cancer cell lines to predict dependency of each patient sample.

In addition, applying single cell transcriptome data to the model for predicting cell survival dependency makes it possible to identify a dependency pattern of respective cells in tumor heterogeneity, and genes showing the same dependency among several cells may be selected as universal dependency genes. Genes that are essential for survival of cancer cells and homogeneously expressed at an individual cell level may be selected as dependency genes that may serve as effective diagnostic and/or therapeutic targets.

The model for predicting cell survival dependency is a model based on neural networks of deep learning consisting of an input layer, multiple hidden layers, and an output layer. The neural networks are configured in a way that, when the aforementioned *in vitro* data are input into the input layer, the relationship between gene expression pattern in a cell and the cell apoptosis is learned in the one or more hidden layers, and then a predetermined probability value is output in the output layer. Here, the predetermined probability value includes a value indicating a probability of cell apoptosis and a value indicating a probability of cell growth.

When the single cell transcriptome data on cancer patients are available, the cancer patient-specific dependency genes can be identified by using the model for predicting cell survival dependency. Otherwise, the universal dependency genes can be identified by using other published data on cancer patient samples.

## Example 2. Selection of neoantigens derived from universal dependency gene and significance thereof as diagnostic/therapeutic target

A neoantigen refers to a peptide or a protein fragment that binds to an MHC protein and is presented on a surface of a cancer cell, and thus is recognized as an antigen by immune cells, which is not present in a normal cell, but is generated by cancer-specific mutations. The neoantigen is a key element of immunotherapy, and it is known that the greater the number of neoantigens, the better the responsiveness to immunotherapy. In this regard, a neoantigen load is utilized as a diagnostic marker. However, since neoantigens are derived from various genes and have different characteristics, usefulness of the neoantigens as diagnostic markers or therapeutic targets will vary. In this Example, as described in Example 1, neoantigens derived from genes on which cancer cell survival is dependent or essential for cancer cell survival were selected as useful neoantigens capable of maximizing effects of immunotherapy, and the selected neoantigens were found responsive to immunotherapy.

Specifically, the responsiveness to immunotherapy was compared by employing cohorts treated with immune checkpoint blockades listed in Table 1.

Table 1

| Cohort name | Tumor type | Cohort size | Data availability | Reference |
|---|---|---|---|---|
| SMC | Lung cancer | 122 | Pre-therapy | |
| Rizvi | Lung cancer | 34 | Pre-therapy | Science 348, 124-128 |
| Hellmann | Lung cancer | 75 | Pre-therapy | Cancer Cell 33, 843-852 |
| Hugo | Melanoma | 38 | Pre-therapy | Cell 165, 35-44 |
| Van Allen | Melanoma | 110 | Pre-therapy | Science 350, 207-211 |
| Snyder | Melanoma | 64 | Pre-therapy | N. Engl. J. Med. 371, 2189-2199 |
| Riaz | Melanoma | 68 | Pre-/On-therapy | Cell 171, 934-949 |

## 2-1. Pre-therapy cohort

By using the *in vitro* dependency data and expression homogeneity data of the published lung cancer and melanoma cohorts with only pre-therapy results, the usefulness of the screening method for the neoantigen based on the cancer cell survival dependency according to an embodiment of the present disclosure was investigated. The analysis results are shown in FIG. 5. Specifically, the genes from which the neoantigens were derived were aligned according to the essentiality for the cancer cell survival, top 500 to 2000 (high dependency) genes and bottom 500 to 2000 (low dependency) genes were selected, and the differential neoantigen load between patients with good prognosis and patient with poor prognosis for immunotherapy was calculated according to Equation 1

$$\text{Differential neoantigen load} = \frac{\text{mean}_{responder} - \text{mean}_{nonresponder}}{\text{mean}_{all\_patients}}$$

It can be considered the larger the difference the better explanatory power for the treatment prognosis. Here, the gray dotted line indicates a difference in the number of neoantigens calculated for all genes according to an existing standard method. As a result, it was found that use of a small number of genes of high essentiality or expression homogeneity provides better explanatory power than use of all genes, while use of genes having low essentiality or expression heterogeneity fails to provide good explanatory power. Similarly, the differential neoantigen load was calculated according to expression homogeneity and gene expression level. Accordingly, it was found that the essentiality and expression homogeneity of a gene provide better explanatory power for the treatment prognosis than the expression level of the gene. Specifically, the genes from which the neoantigens were derived were aligned according to the essentiality for the cancer cell survival, top 500 to 2000 (high dependency) genes and bottom 500 to 2000 (low dependency) genes were selected, and the differential neoantigen load between patients with good prognosis and patient with poor prognosis for immunotherapy was calculated for each gene. The larger difference is considered as having better explanatory power for the treatment prognosis. Here, the gray dotted line indicates a difference in the number of neoantigens calculated for all genes according to an existing standard method. As a result, it was found that use of a few genes having high essentiality or expression homogeneity provides better explanatory power than use of all genes, while use of genes having low essentiality or expression heterogeneity provides poor explanatory power. Similarly, the differential neoantigen load was calculated according to expression homogeneity and gene expression level. Overall, it was found that the essentiality and expression homogeneity of a gene provide better explanatory power for the treatment prognosis than the expression level of the gene.

These results suggest that the immune evasion may occur more actively when the neoantigens subject to immune response are derived from the genes not essential for the proliferation or survival of cancer.

FIG. 6 is a schematic diagram of the immune evasion mechanism of cancer cells.

### 2-2. Pre-therapy and on-therapy cohorts

The dependency of genes from which the neoantigens were derived and the immunotherapy responsiveness were analyzed by using the Riaz cohort data including both pre-therapy and on-therapy data of the immune checkpoint blockades.

The analysis results are shown in FIG. 7. Specifically, it shows the results of comparing clonal changes and gene expression changes before and after the treatment in the cohort treated with the melanoma checkpoint blockade (Riaz) by the essentiality of the function of the gene from which the neoantigen is derived for the cancer survival (high dependency vs. low dependency) and the homogeneity of the gene expression (homogenous vs. heterogenous expression).

Here, CR/PR and SD/PD refer to positive prognosis and negative prognosis, respectively. Accordingly, it was found that in the patient group with a good treatment response (CR(complete remission)/PR(partial remission)), neoantigens derived from genes having expression homogeneity and genes essential for survival of cancer cells were mainly targeted for an anticancer immune reaction, thereby leading to clonal contraction and reduction in the expression level at the RNA level. Meanwhile, it was found that, in the patient group with a poor treatment response (SD(stable disease) and PD(progressive disease)), neoantigens derived from genes having expression heterogeneity and genes not essential for survival of cancer cells were the main targets of immune attack, thereby leading to reduced gene expression and successful immune evasion through immunoediting, and thus the clonal expansion.

### 2-3. MSK-Integrated Mutation Profiling of Actionable Cancer Targets (IMPACT)

MSK-IMPACT was used to verify the dependency of genes from which neoantigens were derived and immunotherapy responsiveness for various carcinomas.

In the previous study, for 468 genes included on the so-called Memorial Sloan Kettering Cancer Center (MSKCC) panel, a mutation load (proportional to the neoantigen load) in 1,662 people who received the checkpoint blockade treatment was measured over various cancer types, and the mutation load was found to be an important determinant of the immunotherapy responsiveness (Nat. Genet. 51:202-206, 2019).

In this Example, it was found that use of top 50 % of 468 genes present on the MSKCC panel in terms of essentiality for cancer cell survival (High dependency) to measure a mutation load resulted in a higher association with the responsiveness of the immune checkpoint blockade treatment than use of all 468 genes.

The results are shown in the upper panel in FIG. 8. The results were obtained by performing survival analysis for the the entire MSKCC panel (All) or the top 50 % (High fitness) or bottom 50 % (Low fitness) of the genes having essentiality for the cancer cell survival and compared post treatment survival probability with the hazard ratio (HR) values and p-values shown, respectively. The low HR values and low p-values indicate that the corresponding gene group has high explanatory power for the treatment prognosis. Accordingly, the mutation load of 226 genes belonging to the top 50 % of the genes having high dependency on cancer cell survival was more highly associated with the immunotherapy responsiveness than the mutation load of all 486 genes. In addition, the middle and bottom panels of FIG. 8 show the results of analyzing the published immunotherapy cohorts of lung cancer and melanoma, respectively, for all genes or top 500 genes and bottom 500 genes among the genes having essentiality for the cancer cell survival.

For each gene group, the patients were grouped into patients having high mutation burden or neoantigen load and patients having low mutation burden or neoantigen load and compared the post-treatment survival probability with HR values and p-values shown for each group. As a result, it was confirmed that the treatment prognosis was better explained by a small number of high dependency genes for cancer cell survival than all genes according to the standard method. Taken together, it was confirmed that the essentiality of genes from which antigens (including neoantigens and surface antigens) were derived, for cancer cells and the expression homogeneity in each cell in a tissue significantly affect the immune responsiveness of cancer.

It was also found that it is essential to make treatment targeted to neoantigens derived from genes essential for the cancer survival and genes homogeneously expressed, in order to minimize the immune evasion of cancer, and that the neoantigen load may be used as markers for prediction of immunotherapy prognosis.

### Example 3. Selection of neoantigen derived from cancer patient-specific dependency gene and significance of the neoantigen as diagnostic/therapeutic target

In this Example, data obtained from a specific cancer patient sample was applied to a model for predicting cell survival dependency to obtain neoantigens derived from selected cancer patient-specific dependency genes, and then association between the neoantigens and the survival of cancer patients was investigated.

In Example 2, the association between the neoantigens derived from universal dependency gene and the survival of patients treated with immunotherapy was investigated.

The dependency data used in Example 2 were derived from the *in vitro* cancer cell line experiment described in Example 1, and accordingly, genes having universal dependency in several cancer cell lines, rather than dependency in a specific patient, were identified. As described above, a model for predicting cell survival dependency for predicting the cell survival dependency on gene expression may be generated by learning the relationship between the patterns of gene expression levels and the cell apoptosis based on the *in vitro* dependency data. When the gene expression profile of a cancer patient is input to the model, cancer patient-specific dependency genes may be identified. Then, the transcriptome data for lung cancer patients and breast cancer patients published by The Cancer Genome Atlas (TCGA) were used to find whether neoantigens obtained based on the cancer patient-specific *in silico* dependency data can serve as diagnostic/therapeutic targets of significance (https://portal.gdc.cancer.gov/). Since these patients did not actually receive the immunotherapy, the patients were divided into a sample having high penetration of immune cells (referred to as high leukocyte fraction) and a sample having low penetration of immune cells (referred to as low leukocyte fraction), assuming that an effect similar to immunotherapy would be seen in the sample having high penetration of immune cells. The results are shown in FIG. 9.

The number of neoantigens derived from the cancer patient-specific genes essential for the proliferation or survival of cancer cells (High dependency) (shown in a black bar graph in FIG. 9) was found to have better explanatory power than the total number of neoantigens (shown in horizontal line in FIG. 9) or the number of neoantigens derived from genes not essential for the survival of cancer cells (Low dependency) (shown in a gray bar graph in FIG. 9). In particular, these results were observed only in the samples with high penetration of immune cells, and the cancer patient-specific dependency data was found to generally show a greater difference in the explanatory power than the universal dependency data.

## Example 4. Model for predicting binding affinity between neoantigen and antigen-presenting cell

For a neoantigen to be responsive to immunotherapy, the neoantigen must be processed by an antigen-presenting cell to be bound to HLA on a surface of the antigen-presenting cell.

Benchmark datasets from the Immune Epitope Database (IEDB) used in previous studies were employed to build and validate a model for predicting binding affinity between the neoantigen and the antigen-presenting cell and the results from the model were compared with the prediction power results from the existing machine learning algorithms published by the IEDB.

Here, the model for predicting binding affinity between the neoantigen and the antigen-presenting cell is a CNN model that includsmultiple convolutional layers, a full connected layer, and an output layer, but nopooling layer so that all output values of the multiple convolution layers are used for prediction.

The multiple convolutional layers extract interaction features from input data which are map data including parameters indicating binding affinity between amino acids of a peptide and amino acids of HLA.

The multiple convolutional layers may perform convolution by using a specific number of kernels or weight matrices. The fully connected layer receives and integrates the output values of the multiple convolutional layers as an input, and the output layer generate information about the binding probability by using a sigmoid function. FIG. 10 is a schematic diagram of a model for predicting binding affinity of the neoantigen and MHC used in the neoantigen screening method according to an embodiment of the present disclosure (CNN-MHC). The built model was learned by using the results of 50,000 or more peptide-MHC *in vitro* binding experiments available in the IEDB was tested for performance with the test data set updated by the IEDB every week. For more than 70 % of the test data set, the model was found to outperform the most widely used NetMHCpan as well as the existing algorithms, such as SMMPMBEC, ANN, and NetMHCcon.

The results are shown in FIG. 11.

## 4-1. Confirmation of binding of neoantigen to HLA

To confirm whether the neoantigen actually binds to the HLA molecule as predicted by the model (CNN-MHC) according to this Example, the binding ability of the neoantigen to the HLA was tested.

Specifically, the binding ability of peptides predicted to bind to HLA-A02 was analyzed by using a T2 cell line expressing the HLA-A02 (ATCC CRL-1992). The peptides predicted to bind to the HLA-A02 are shown in Table 2 (SEQ ID NOs: 1 to 50). In Table 2, CNN-MHC values are obtained from a deep learning model built based on the experimental values that converted the binding strength between respective amino acids of the neoantigen and the MHC into a matrix form, and mean a value converted to a probability between 0 and 1. NetMHC values refer to predicted values of the binding between the MHC protein and the peptide as calculated with NetMHCPan-4.1, which is a model learned based on mass spectrometry data with the latest version of the NetMHC tool commonly used for the binding prediction of the neoantigens. It is considered that the higher these two values, the higherthe binding probability. Specifically, a culture of T2 cell line ($1\times10^6$/ml) was treated with a peptide at a concentration of 50 µg/ml for 24 hours, and then stained with APC-labeled HLA-A2 monoclonal antibody. The resulting cells were analyzed by flow cytometry to find the binding ability thereof. Here, DMSO was used as a negative control, and Mart-1 and NY-ESO were used as positive controls.

Table 2

| Mutation information | | | Predicted neoantigen amino acid sequence | CNN-MHC value | NetMHC value |
|---|---|---|---|---|---|
| KMT2D | p.P2354del | inframe_deletion | RLWHLLLQV | 0.9808 | 0.9425 |
| APC | p.K1308_E1309del | inframe_deletion | LMFSRCTSV | 0.9641 | 0.4598 |
| NOTCH1 | p.P1228del | inframe_deletion | RLLDEYNLV | 0.9873 | 0.9336 |
| NOTCH2 | p.C19W | missense_variant | LLALWLCWA | 0.9684 | 0.0357 |
| TP53 | p.P89del | inframe_deletion | HLYPGPCHL | 0.9355 | 0.9093 |
| KMT2D | p.G1235del | inframe_deletion | RLMTHYCAM | 0.9720 | 0.0529 |
| KMT2D | p.P1460Z | inframe_deletion | GLLPLASTV | 0.9721 | 0.8932 |
| TP53 | p.R110L | missense_variant | FLLGFLHSG | 0.9706 | 0.1621 |
| ARID1A | p.Y1324del | inframe_deletion | LMNSLVSQV | 0.9619 | 0.6929 |
| ARID1A | p.G436del | inframe_deletion | CLYAFECKI | 0.9639 | 0.0264 |
| TP53 | p.C141F | missense_variant | KTFPVQLWV | 0.9224 | 0.6665 |
| ARID1A | p.F799del | inframe_deletion | GLLAESTWA | 0.9132 | 0.1453 |
| ARID1A | p.F799del | inframe_deletion | SMQNHIPQV | 0.9390 | 0.9747 |
| TP53 | p.P89del | inframe_deletion | CQLAKTCPV | 0.9728 | 0.0177 |
| TP53 | p.R267P | missense_variant | LLGPNSFEV | 0.9699 | 0.9425 |
| CTCF | p.N314del | inframe_deletion | SLSYWRASV | 0.9863 | 0.2118 |
| PTEN | p.D92V | missense_variant | RVAQYPFEV | 0.9650 | 0.8458 |
| ACVR2A | p.K435del | inframe_deletion | LLAETCWNG | 0.8807 | 0.0036 |
| NCOR1 | p.K528del | inframe_deletion | VLQPAPHQV | 0.8670 | 0.9844 |

(continued)

| Mutation information | | | Predicted neoantigen amino acid sequence | CNN-MHC value | NetMHC value |
|---|---|---|---|---|---|
| CHD8 | p.K584del | inframe_deletion | YLHCEWATI | 0.9392 | 0.1572 |
| ARID1A | p.F799del | inframe_deletion | LLAESTWAL | 0.9951 | 0.9478 |
| TRIP12 | p.S1195del | inframe_deletion | PLEEWNQWV | 0.9247 | 0.0091 |
| TP53 | p.K132E | missense_variant | ALNEMFCQL | 0.9589 | 0.7345 |
| TP53 | p.G266R | missense_variant | LLRRNSFEV | 0.8559 | 0.1652 |
| TP53 | p.P190L | missense_variant | ALPQHLIRV | 0.8962 | 0.9346 |
| MAP3K1 | p.S1330W | missense_variant | MAGGWVAHL | 0.8525 | 0.0262 |
| TP53 | p.R267L | missense_variant | GLNSFEVRV | 0.9465 | 0.8750 |
| TP53 | p.C141F | missense_variant | CQLAKTFPV | 0.9771 | 0.1100 |
| ARID1A | p.G436del | inframe_deletion | NMANMPPQV | 0.8712 | 0.8093 |
| CHD8 | p.K584del | inframe_deletion | YTEAEEFFV | 0.8758 | 0.0640 |
| CDH1 | p.R124del | inframe_deletion | TLMSVPRYL | 0.9526 | 0.8410 |
| ARID1A | p.G436del | inframe_deletion | KMWVDRYLA | 0.8730 | 0.1684 |
| TP53 | p.H193Y | missense_variant | GLAPPQYLI | 0.9391 | 0.7980 |
| TP53 | p.P89del | inframe_deletion | FLINSSYPG | 0.9759 | 0.0100 |
| PTEN | p.P246L | missense_variant | FMYFEFPQL | 0.9757 | 0.8519 |
| NOTCH1 | p.P1228del | inframe_deletion | AAFVLLFFV | 0.9125 | 0.0420 |
| CDH1 | p.R124del | inframe_deletion | ILHVAVTNV | 0.8158 | 0.7004 |
| KMT2D | p.Q773del | inframe_deletion | RLRSRTCLL | 0.7666 | 0.0248 |
| ARID1A | p.F799del | inframe_deletion | ALDTINILL | 0.8745 | 0.9349 |
| APC | p.G2191del | inframe_deletion | LIYQMAPAV | 0.7720 | 0.2204 |

(continued)

| Mutation information | | | Predicted neoantigen amino acid sequence | CNN-MHC value | NetMHC value |
|---|---|---|---|---|---|
| ARID1A | p.F799del | inframe_ deletion | SQLPGLLEL | 0.8539 | 0.8652 |
| TP53 | p.C141Y | missense_ variant | CQLAKTYPV | 0.9774 | 0.0972 |
| ARID1B | p.P661del | inframe_ deletion | GMGPPMPTV | 0.7779 | 0.9516 |
| CDH1 | p.R124del | inframe_ deletion | FTINRNTGV | 0.9074 | 0.1951 |
| EP300 | p.A1629V | missense_ variant | LMDGRDVFL | 0.8355 | 0.7187 |
| NOTCH1 | p.P1228del | inframe_ deletion | PLIEEACEL | 0.8883 | 0.0834 |
| ARID1A | p.G436del | inframe_ deletion | TMYQQQQQV | 0.7810 | 0.8726 |
| NOTCH1 | p.P1228del | inframe_ deletion | SALHWAAAV | 0.8763 | 0.0472 |
| NOTCH1 | p.P1228del | inframe_ deletion | RMHDGTTPL | 0.8935 | 0.7249 |
| CDK12 | p.W1450del | inframe_ deletion | LLMENSIGG | 0.7974 | 0.0102 |

FIG. 12 shows the results of the MHC binding analysis.

80 % of the peptides predicted by the model (CNN-MHC) of this Example were actually found to bind to the HLA-A02. Compared with the widely used prediction values of NetMHCpan-4.1 (http://www.cbs.dtu.dk/services/NetMHCpan-4.1/), the model of this Example was found to have better prediction power. Meanwhile, the aforementioned Examples may be performed by a processor 120 executing at least one instruction stored in a memory 110 of a system shown in FIG. 3.

Referring to FIG. 4, the processor 120 includes: a data acquisition unit 121 that obtains sequencing data of exomes, transcriptomes, or the entire genome; a prediction model generation unit 122 that generates a model for predicting cell survival dependency and a model for predicting binding affinity; a gene identification unit 123 that identifies a gene on which cancer cell survival is dependent by using the model for predicting cell survival dependency; and a neoantigen selection unit 124 that collects neoantigens from a gene expression profile of a cancer patient and selects a neoantigen having binding affinity to HLA by using a model for predicting binding affinity. As the prediction model generation unit 122 repeatedly executes instructions, the model for predicting cell survival dependency and the model for predicting binding affinity may be updated based on new input data.

Prediction models generated and updated by the prediction model generation unit 122 may be stored in a memory.

**Example 5. Anti-cancer vaccine containing neoantigen**

As described in the aforementioned Examples, an anti-cancer vaccine containing a neoantigen derived from a gene essential for cancer cell survival may be prepared.

By performing exome sequencing on a mouse cell line with cancer cells or tissues transplanted, mutations not found in normal cells may be discovered, and these mutations may be applied to the model for predicting binding affinity to antigen-presenting cells to select candidate neoantigens.

Genes from which the neoantigens were derived are subjected to the model for predicting cell survival dependency to determine the essentiality for the cancer cell survival based on the dependency data on the corresponding cancer, and the neoantigens are aligned according to the essentiality to select top 5 and bottom 5 neoantigens. All possible peptide sequences including any of the selected neoantigens in 9 to 30 amino acids long are generated and tested to select sequences having chemical properties suitable for peptide synthesis, e.g., high hydrophilicity (Kyte-Doolittle GRAVY < 0) and having low instability index (InstaIndex < 40). Then, peptides having the selected sequences may be synthesized, and used to prepare an anti-cancer vaccine containing the neoantigens.

## 5-1. Selection of neoantigen

The exome and transcriptome information of cancer cell lines was analyzed by using the model for predicting cell survival dependency and the model for predicting binding affinity between the neoantigen and the antigen-presenting cell as described in Examples 1 to 4, and accordingly, mutations derived from the dependency gene having high cell survival essentiality and having HLA-binding ability were selected.

## 5-2. Confirmation of immune response by neoantigen

To confirm whether the neoantigen induces immune response thereto, a test to detect T cells that recognize the neoantigen peptides with predicted or experimentally verified HLA-binding ability including the neoantigen peptides selected in 5-1 was conducted.

Specifically, mice transplanted with a mouse cancer cell line were used to identify a T cell pool having specificity. $1 \times 10^6$ LLC-1 (ATCC CRL-1642), a mouse cancer cell line were subcutaneously injected into the flank of a 6-week-old male C57BL/6 mouse weighing 20 g to generate a mouse model. Specifically, through the analysis of exomes and transcriptomes of the cancer cell line, 15 mutations predicted to have high essentiality for cell survival and high binding force to the antigen-presenting cell were selected as a neoantigen group expected to induce binding to HLA and reactive CD8+ T cells.

The information on the selected peptides is summarized in Table 3 (SEQ ID NOs: 51 to 65). The CNN-MHC values listed in Table 3 are values obtained by building a deep learning model based on experimental values that converted the binding strength between respective amino acids of the neoantigen and the MHC into a matrix form, and are values converted into a probability value between 0 and 1. Here, a higher value indicates a higher probability of binding. To determine the reaction of CD8+ T cells to the neoantigens, the ELISpot analysis based on IFN$\gamma$ secretion was performed by using spleen cells extracted from the spleen of mice vaccinated with 9-mer or 15-mer peptides synthesized with the mutation sequences. 10 peptides (66.7 %) out of 15 candidate neoantigen peptides with expected reactivity were found to induce IFN$\gamma$ secretion from T cells. The results are shown in FIG. 13. Accordingly, it was confirmed that the neoantigens selected by using the method according to an embodiment of the present disclosure actually bound to the HLA and effectively induced an immune response.

Table 3

| Mutation Information | | | HLA type | Predicted neoantigen amino acid sequence | CNN-MHC value |
|---|---|---|---|---|---|
| Twistnb | p.K229_K230insK | inframe_ insertion | H2-IEd | TWWEEVVEKTPKKKK | 0.9999 |
| Las1l | p.T404_S405insS | inframe_ insertion | H2-IEd | STGKAPYTLDTLHED | 0.9898 |
| Irx1 | p.S447W | missense_ variant | H2-IAd | ALPERDLVTRPDWPP | 0.9830 |
| Muc5b | p.I2569N | missense_ variant | H2-IAd | GATTMSVNISTIGTN | 0.9736 |
| Smarca5 | p.M221R | missense_ variant | H2-IAd | LGKTLQTISLLGYRK | 0.9538 |
| Eri1 | p.E136K | missense_ variant | H2-IAd | YDYICIIDFEATCKE | 0.9350 |
| Tph2 | p.A434V | missense_ variant | H2-IAd | FVKSITRPFSVYFNR | 0.9229 |
| Eef2 | p.A163fs | frameshift | H2-Dd | RAPAGAAAG | 0.9161 |

(continued)

| Mutation Information | | | HLA type | Predicted neoantigen amino acid sequence | CNN-MHC value |
|---|---|---|---|---|---|
| Ssrp1 | p.K623E | missense_ variant | H2-IAd | GRGDSSERDKSKKKK | 0.9158 |
| Oas1g | p.R74K | missense_ variant | H2-IAd | GKSDADLWFLNNLT | 0.8983 |
| Gm15557 | p.L116V | missense_ variant | H2-IAd | SRHSCAEFVKFRKSF | 0.8961 |
| Ppp1cc | p.F227S | missense_ variant | H2-IAd | SFTSGAEVVAKFLHK | 0.8916 |
| Pdcd6ip | p.L187R | missense_ variant | H2-IAd | TVGTRSLIMLAQAQE | 0.8574 |
| Dync1h1 | p.N2529S | missense_ variant | H2-IAd | PTAPSVPIIDYEVSI | 0.8228 |
| Eef2 | p.T141I | missense_ variant | H2-IAd | SGVCVQTEIVLRQAI | 0.7846 |

**Example 6. Prediction of treatment prognosis for cancer patient by using neoantigen**

As described in the aforementioned Examples, neoantigens derived from a gene essential for cancer cell survival may be used to predict treatment prognosis for a cancer patient.

By performing exome sequencing on a patient sample, mutations not found in normal cells but found only in cancer cells are identified, and these mutations may be applied to the model for predicting binding affinity with antigen-presenting cells to select candidate neoantigens.

The model for predicting cell survival dependency is used to determine the essentiality of the genes from which the neoantigens were derived, for the cancer cell survival based on the dependency data on the corresponding cancer, and the neoantigens are aligned according to the essentiality for survival to select the same number of genes (e.g., 500 genes) from the top and the bottom. The patients treated with immunotherapy are divided into a patient group who responded to the immunotherapy and a patient group who did not respond to the immunotherapy. Then, the patient groups are compared in terms of the number of neoantigens derived from the genes selected from the top (i.e., highest essentility) and that from the genes selected from the bottom (i.e., lowest essentiality) to determine whether the load of neoantigens derived from the genes of high essentiality is highly associated with the treatment prognosis of a patient. The determination of the association between the number of neoantigens and the treatment prognosis of patients are reiterated with change in the number of genes selected, thereby choosing the number of neoantigens derived from the dependency gene necessary to predict the treatment prognosis of the patient. FIG. 8 shows that the mutation burden or the neoantigen load derived from the genes having high essentiality for the cancer cell survival provides high explanatory power and can be used to predict the treatment prognosis of the cancer patient.

The foregoing descriptions are only for illustrating the present disclosure, and it will be apparent to a person having ordinary skill in the art to which the present invention pertains that the embodiments disclosed herein can be easily modified into other specific forms without changing the technical principle or essential features.

Therefore, it should be understood that Examples described herein are illustrative in all respects and are not limiting. For example, each component described as in a single form may be implemented in a distributed manner, and likewise components described as being distributed may be implemented in a combined form.

**Claims**

1. A method of screening neoantigens, the method comprising:

obtaining sequencing data of an exome, a transcriptome, a single cell transcriptome, a peptidome, or an entire genome from a cancer patient;
identifying genes essential for cancer cell survival; and

obtaining neoantigens derived from the genes essential for cancer cell survival.

2. The method of claim 1, further comprising determining binding affinity of the neoantigens to HLA of an antigen-presenting cell.

3. The method of claim 1, wherein the identifying of the genes essential for cancer cell survival comprises identifying genes essential for cancer cell survival by using a model for predicting cell survival dependency, wherein the model for predicting cell survival dependency is generated by learning a relationship between gene expression in a cell and cell apoptosis, and identifies as the gene essential for cancer cell survival, a gene of which expression reduction or removal causes cell apoptosis.

4. The method of claim 3, wherein the identified gene essential for cancer cell survival causes cancer cell apoptosis when its expression level is decreased or removed, but does not affect survival of a normal cell.

5. The method of claim 3, wherein the relationship between gene expression in a cell and cell apoptosis is based on *in vitro* data or *in silico* data on cancer cell line apoptosis according to a reduction in expression or removal of a targeted gene.

6. The method of claim 3, wherein the identifying of the gene essential for cancer cell survival further comprises determining whether the gene essential for cancer cell survival identified by using the model for predicting cell survival dependency is homogeneously expressed in all cancer cells obtained from a cancer patient.

7. The method of claim 1, wherein the obtaining of the neoantigens derived from the gene essential for cancer cell survival comprises: obtaining neoantigens of a cancer patient by comparing a sequence of a cancer cell and a sequence of a normal cell, based on sequencing data obtained from the cancer patient; and collecting neoantigens derived from the gene essential for cancer cell survival from the obtained neoantigens.

8. The method of claim 7, wherein the collecting of the neoantigens further comprises selecting nonsynonymous mutations in the genes essential for cancer cell survival.

9. The method of claim 1, wherein the neoantigen is specific to the cancer patient.

10. The method of claim 2, wherein the determining of binding affinity of the neoantigens to HLA of an antigen-presenting cell comprises obtaining prediction of the binding affinity by inputting a sequence of the neoantigen to a model for predicting binding affinity of a peptide to HLA of an antigen-presenting cell, and
the model for predicting binding affinity of a peptide is generated by learning data regarding interaction between amino acids of a peptide and amino acids of an HLA.

11. The method of claim 10, wherein the antigen-presenting cell comprises a dendritic cell, a macrophage, a B cell, or a combination thereof.

12. The method of claim 10, wherein the HLA is MHC class I or MCH class II.

13. The method of claim 10, wherein the neoantigen is determined to have binding affinity to the HLA, when a CNN-MHC value between the neoantigen and the HLA of the antigen-presenting cell is > 0.5.

14. A system for screening neoantigens, the system comprising:

a memory for storing at least one instruction; and
at least one processor for executing the at least one instruction stored in the memory,
wherein the at least one processor executes the at least one instruction
to generate a model for predicting cell survival dependency that predicts cell survival dependency on gene expression by learning a relationship between gene expression level in a cell and cell apoptosis,
to identify a gene essential for cancer cell survival by inputting a gene expression profile of a cancer patient to the model for predicting cell survival dependency, and
to obtain from the gene expression profile of a cancer patient a neoantigen derived from the gene essential for cancer cell survival,
to generate a model for predicting binding affinity of a neoantigen which predicts binding affinity based on amino acid interactions between a peptide and an antigen-presenting cell, and
to select a neoantigen having binding affinity to HLA of the antigen-presenting cell by using the model for predicting binding affinity of a neoantigen.

15. The system of claim 14, wherein the model for predicting cell survival dependency is generated from learning of relationship between gene expression in a cell and cell apoptosis, and identifies as a gene essential for cancer cell survival, a gene of which expression reduction or removal causes cancer cell apoptosis.

16. The system of claim 14, wherein the at least one processor executes the at least one instruction to select a neoantigen having binding affinity when a CNN-MHC value between the neoantigen and the HLA of the antigen-presenting cell is > 0.5.

17. The system of claim 14, wherein the at least one processor executes the at least one instruction to learn relationship between gene expression and cell apoptosis, and relationship between a neoantigen and HLA of an antigen-presenting cell for binding affinity, respectively.

18. The system of claim 17, wherein the relationship between gene expression in a cell and cell apoptosis is based on *in vitro* data or *in silico* data on cancer cell apoptosis according to a reduction in expression or removal of a targeted gene.

19. The system of claim 14, wherein the model for predicting binding affinity of the neoantigen is generated from learning of data regarding interaction between amino acids of the peptide and amino acids of the HLA.

20. The system of claim 14, wherein the gene expression profile of a cancer patient is sequencing data of an exome, a transcriptome, a single cell transcriptome, a peptidome, or an entire genome.

21. A method of preparing an anti-cancer vaccine, the method comprising:

   obtaining a neoantigen by the method of any one of claims 1 to 13; and
   preparing an anti-cancer vaccine comprising the neoantigen.

22. The method of claim 21, further comprising: obtaining peptide sequences comprising the neoantigen, the peptide sequences consisting of 9 to 30 amino acids; and
selecting a peptide sequence having hydrophilicity and stability from the obtained peptide sequences.

23. The method of claim 22, wherein the selected peptide sequence has Kyte-Doolittle GRAVY < 0 and InstaIndex < 40.

24. An anti-cancer vaccine comprising a neoantigen obtained by the method of any one of claims 1 to 13.

25. A method of providing information to predict treatment prognosis of a cancer patient, the method comprising:

   obtaining a neoantigen by the method of any one of claims 1 to 13; and
   measuring the neoantigen load in a sample from the cancer patient.

26. The method of claim 25, further comprising comparing the obtained neoantigen load and a neoantigen load obtained from a control group consisting of cancer patients for whom treatment prognosis have been confirmed.

27. A composition for predicting treatment prognosis of a cancer patient, the composition comprising a neoantigen obtained by the method of any one of claims 1 to 13.

FIG. 1

# FIG. 2

START

Obtaining sequencing data from cancer patient —S10

Identifying gene essential for cancer cell survival —S20

Obtaining neoantigens derived from genes essential for cancer cell survival —S30

Determining binding affinity of neoantigen to antigen-presenting cell —S40

END

# FIG. 3

# FIG. 4

120

**121**

Data acquisition unit

**122**

Prediction model genration unit

**123**

Gene identification unit

**124**

Gene identification unit

FIG. 5

FIG. 6

FIG. 7

# FIG. 8

FIG. 9

FIG. 10

EP 4 116 436 A1

## FIG. 11

FIG. 12

MHC binding assay (increase of ean fluorescence intensity)

Correct
Incorrect

DeepNeo-Pan
NetMHC

MFI Increase (N.C)

# FIG. 13

Detection of IFN-γ secreting splenocytes by ELISPOT assays

## INTERNATIONAL SEARCH REPORT

| International application No. |
| --- |
| **PCT/KR2021/000116** |

| | |
| --- | --- |
| **A. CLASSIFICATION OF SUBJECT MATTER** | |
| **C12Q 1/6886**(2018.01)i; **G16B 35/10**(2019.01)i | |
| According to International Patent Classification (IPC) or to both national classification and IPC | |

**B. FIELDS SEARCHED**

Minimum documentation searched (classification system followed by classification symbols)

C12Q 1/6886(2018.01); A61K 39/00(2006.01); G01N 33/569(2006.01); G16B 50/40(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 신생항원(neoantigen), HLA, 암(cancer), 스크리닝(screening), 면역 회피(immune evasion)

**C. DOCUMENTS CONSIDERED TO BE RELEVANT**

| Category* | Citation of document, with indication, where appropriate, of the relevant passages | Relevant to claim No. |
| --- | --- | --- |
| Y | GARCIA-GARIJO, A. et al. Determinants for neoantigen identification, Frontiers in immunology. June 2019, vol. 10, thesis no.: 1392, pp. 1-19. See abstract; figures 1-2; table 3; and pages 2-4. | 1-27 |
| Y | APAOLAZA, I. et al. An in-silico approach to predict and exploit synthetic lethality in cancer metabolism. Nature communications. 2017, vol. 8, thesis no.: 459, pp. 1-9. See abstract; figure 1; and pages 1-2 and 6. | 1-27 |
| Y | KR 10-2018-0081490 A (CANCER RESEARCH TECHNOLOGY LIMITED) 16 July 2018 (2018-07-16) See paragraph [0015]; and claim 3. | 25-27 |
| A | US 2017-0202939 A1 (WASHINGTON UNIVERSITY) 20 July 2017 (2017-07-20) See abstract; and claim 14. | 1-27 |
| A | KR 10-2019-0140935 A (GRITSTONE ONCOLOGY, INC.) 20 December 2019 (2019-12-20) See abstract; and claims 1-33. | 1-27 |

| ☐ Further documents are listed in the continuation of Box C. | ☑ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 April 2021** | **27 April 2021** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/000116**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/KR2021/000116**

| Patent document cited in search report | | | Publication date (day/month/year) | Patent family member(s) | | | Publication date (day/month/year) |
|---|---|---|---|---|---|---|---|
| KR | 10-2018-0081490 | A | 16 July 2018 | AU | 2016-319316 | A1 | 22 February 2018 |
| | | | | CA | 2997651 | A1 | 16 March 2017 |
| | | | | CN | 108135985 | A | 08 June 2018 |
| | | | | EP | 3347039 | A1 | 18 July 2018 |
| | | | | JP | 2018-527935 | A | 27 September 2018 |
| | | | | RU | 2018112516 | A | 10 October 2019 |
| | | | | RU | 2018112516 | A3 | 10 February 2020 |
| | | | | RU | 2739036 | C2 | 21 December 2020 |
| | | | | US | 2018-0251553 | A1 | 06 September 2018 |
| | | | | WO | 2017-042394 | A1 | 16 March 2017 |
| US | 2017-0202939 | A1 | 20 July 2017 | AU | 2015-314776 | A1 | 06 April 2017 |
| | | | | CA | 2961179 | A1 | 17 March 2016 |
| | | | | EP | 3193892 | A1 | 26 July 2017 |
| | | | | EP | 3193892 | A4 | 12 September 2018 |
| | | | | WO | 2016-040900 | A1 | 17 March 2016 |
| KR | 10-2019-0140935 | A | 20 December 2019 | AU | 2018-254526 | A1 | 14 November 2019 |
| | | | | BR | 112019021782 | A2 | 18 August 2020 |
| | | | | CA | 3060569 | A1 | 25 October 2018 |
| | | | | CN | 110636852 | A | 31 December 2019 |
| | | | | CO | 2019012345 | A2 | 17 January 2020 |
| | | | | EP | 3612965 | A1 | 26 February 2020 |
| | | | | JP | 2020-519246 | A | 02 July 2020 |
| | | | | MX | 2019012433 | A | 11 December 2019 |
| | | | | SG | 11201909652 | A | 28 November 2019 |
| | | | | WO | 2018-195357 | A1 | 25 October 2018 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20180107102

**Non-patent literature cited in the description**

- *Science,* vol. 348, 124-128
- *Cancer Cell,* vol. 33, 843-852
- *Cell,* vol. 165, 35-44
- *Science,* vol. 350, 207-211
- *N. Engl. J. Med.,* vol. 371, 2189-2199
- *Cell,* vol. 171, 934-949
- *Nat. Genet.,* 2019, vol. 51, 202-206